# FASCICULE DE BREVET EUROPEEN

(11) **EP 1 265 815 B1**
(45) Date de publication et mention de la délivrance du brevet: **02.02.2005**
(21) Numéro de dépôt: 00983380.7
(22) Date de dépôt: 17.11.2000
(51) Int. Cl.: C01F 17/00, A61K 7/42, C08K 3/22, B01J 13/00

(54) **DISPERSION COLLOIDALE D'UN COMPOSE DE CERIUM ET CONTENANT DU CERIUM III, PROCEDE DE PREPARATION ET UTILISATION**
KOLLOIDALE SUSPENSION EINER CERIUM-III ENTHALTENDEN CERIUMVERBINDUNG, VERFAHREN ZUR DEREN HERSTELLUNG UND DEREN VERWENDUNG
COLLOIDAL DISPERSION OF A CERIUM COMPOUND AND CONTAINING CERIUM III, PREPARATION METHOD AND USE

(30) Priorité: 19.11.1999 FR 9914576
(43) Date de publication de la demande: 18.12.2002
(73) Titulaire: Rhodia Electronics and Catalysis, 17041 La Rochelle Cedex (FR)
(72) Inventeur: CHANE-CHING, Jean-Yves, F-95600 Eaubonne (FR)
(74) Mandataire: Dubruc, Philippe
(86) Numéro de dépôt international: PCT/FR2000/003199
(87) Numéro de publication internationale: WO 2001/036331

(56) Documents cités:
- EP-A- 0 700 870
- EP-A- 0 822 164
- WO-A-79/00248
- US-A- 4 800 072
- DATABASE WPI Section Ch, Week 198929 Derwent Publications Ltd., London, GB; Class A60, AN 1989-210859 XP002141694 & JP 01 148710 A (TAKI CHEMICAL KK), 12 juin 1989 (1989-06-12)

## Description

La présente invention concerne une dispersion colloïdale d'un composé de cérium contenant du cérium III.

Les sols de cérium, tout particulièrement les sols de cérium tétravalent, sont bien connus. Ils présentent un grand intérêt, par exemple pour des applications en cosmétique ou dans le domaine des luminophores. Cependant, dans ces applications, on a besoin de sols qui sont relativement concentrés, stables et purs. Or, les procédés de préparation de tels sols sont complexes; ils nécessitent notamment la m ise en oeuvre de résines échangeuses d'ions. Il serait intéressant de disposer d'une voie d'accès plus facile à de tels sols.

Des sols de cérium tétravalent qui sont préparés par des procédés utilisant des résines sont déjà décrits dans EP 0700870.

JP 01148710 concerne aussi des sols de cérium tétravalent dont le pH est bas du fait de leur procédé de préparation mettant en oeuvre un acide.

WO 079/00248 décrit des dispersions de cérium qui sont colloïdales à des valeurs de pH très acide.

L'objet de l'invention est de résoudre la difficulté évoquée plus haut et donc de permettre d'obtenir des sols purs et suffisamment concentrés selon une voie plus simple.

Dans ce but, l'invention concerne une dispersion colloïdale d'un composé de cérium, caractérisée en ce qu'elle présente une concentration en oxyde de cérium d'au moins 50g/l, un taux de cérium III par rapport au cérium total d'au moins 0,5%, une conductivité d'au plus 5mS/cm, un pH compris entre 4 et 8 et en ce qu'elle est constituée de particules présentant un diamètre moyen d'au plus 15nm.

L'invention concerne aussi un procédé de préparation d'une telle dispersion colloïdale qui est caractérisé en ce qu'on fait réagir au moins un sel de cérium III avec une base et en présence d'un acide en quantité telle que le rapport H⁺/Ce soit supérieur à 1, puis on redisperse dans l'eau le précipité issu de la réaction précédente et on purifie par ultrafiltration la dispersion obtenue.

Le procédé de l'invention n'est pas complexe puisqu'il comporte essentiellement une réaction d'un sel et d'une base qui peut se faire en mettant en oeuvre un appareillage simple. Il part d'un composé de cérium III, cérium III que l'on retrouve en partie dans la dispersion de l'invention, et cette dispersion présente les caractéristiques de pureté recherchées.

D'autres caractéristiques, détails et avantages de l'invention apparaîtront encore plus complètement à la lecture de la description qui va suivre, ainsi que des divers exemples concrets mais non limitatifs destinés à l'illustrer.

Pour la suite de la description, l'expression dispersion colloïdale ou sol d'un composé de cérium désigne tout système constitué de fines particules solides de dimensions colloïdales à base d'oxyde et/ou d'oxyde hydraté (hydroxyde) de cérium, en suspension dans une phase liquide aqueuse, lesdites particules pouvant en outre, éventuellement, contenir des quantités résiduelles d'ions liés ou adsorbés tels que par exemple des nitrates, des acétates, des citrates ou des ammoniums. On notera que dans de telles dispersions, le cérium peut se trouver soit totalement sous la forme de colloïdes, soit simultanément sous la forme d'ions et sous la forme de colloïdes.

Selon une première caractéristique, la dispersion de l'invention présente une concentration d'au moins 50g/l. Elle peut être plus particulièrement d'au moins 100g/l et encore plus particulièrement d'au moins 150g/l. Cette concentration est exprimée en concentration équivalente en oxyde de cérium CeO₂. Elle est déterminée après séchage et calcination sous air d'un volume donné de dispersion.

La seconde caractéristique de la dispersion est son taux de cérium III. Ce taux est d'au moins 0,5%. Ce taux peut être plus particulièrement d'au moins 1% et encore plus particulièrement d'au moins 1,5%. Généralement, il est d'au plus 50%. Il peut être notamment d'au plus 10%. Il est exprimé par le rapport atomique CeIII/Ce total. La dispersion comprend en outre du cérium sous forme cérium IV.

Une troisième caractéristique de la dispersion de l'invention est sa pureté. Cette pureté est mesurée par la conductivité de la dispersion. Cette conductivité est d'au plus 5mS/cm. Elle peut être inférieure à cette valeur et ainsi être, de préférence, d'au plus 2mS/cm et plus particulièrement d'au plus 1 mS/cm. Encore plus particulièrement, elle peut être inférieure à 0,3mS/cm.

Les dispersions de l'invention peuvent présenter en outre un pH élevé, par exemple compris entre 4 et 8. Ces valeurs de pH, voisines de la neutralité, permettent des applications intéressantes des dispersions de l'invention.

Les particules colloïdales qui constituent les sols de l'invention sont fines. Ainsi, elles peuvent présenter un diamètre moyen qui est d'au plus 15nm et qui peut être compris notamment entre 2nm et 15nm, plus particulièrement entre 2nm et 6nm. Ce diamètre est déterminé par comptage photométrique à partir d'une analyse par METHR (Microscopie Electronique par Transmission à Haute Résolution).

Selon une variante préférée, les dispersions de l'invention sont particulièrement pures en anions nitrates. Plus précisément, la teneur en anions nitrates des dispersions, mesurée par la teneur en anions nitrates en poids des particules colloïdales est inférieure à 80ppm.

Enfin, les dispersions selon l'invention peuvent être des dispersions aqueuses, la phase continue étant l'eau, ou des dispersions dans une phase continue qui peut être constituée par un mélange eau/solvant miscible à l'eau ou encore des dispersions dans un solvant organique.

Le solvant organique peut être tout particulièrement un solvant miscible à l'eau. On peut citer, par exemple, les alcools comme le méthanol ou l'éthanol, les glycols comme l'éthylène glycol, les dérivés acétates des glycols comme le monoacétate d'éthylène glycol, les éthers de glycols, les polyols ou les cétones.

Le procédé de préparation des dispersions de l'invention va maintenant être décrit.

Ce procédé comprend une première étape dans laquelle on fait réagir avec une base un sel de cérium III en présence d'un acide.

Comme base, on peut utiliser notamment les produits du type hydroxyde. On peut citer les hydroxydes d'alcalins ou d'alcalino-terreux et l'ammoniaque. On peut aussi utiliser les amines secondaires, tertiaires ou quaternaires. Toutefois, les amines et l'ammoniaque peuvent être préférés dans la mesure où ils diminuent les risques de pollution par les cations alcalins ou alcalino terreux.

Comme sels de cérium III, on peut utiliser plus particulièrement l'acétate, le chlorure ou le nitrate de cérium III ainsi que des mélanges de ces sels comme des mixtes acétate/chlorure.

Selon une caractéristique spécifique du procédé de l'invention, la réaction du sel de cérium avec la base se fait en présence d'un acide.

Comme acides susceptibles d'être utilisés, on peut mentionner les acides minéraux et/ou organiques et plus particulièrement ceux correspondant aux sels de cérium III utilisés dans la réaction. On peut ainsi citer notamment l'acide acétique ou l'acide chlorhydrique.

La quantité d'acide présent ou mis en oeuvre lors de la réaction est telle que le rapport molaire H⁺/Ce soit supérieur à 1, de préférence supérieur à 1,5 et plus particulièrement d'au moins 2. Cette quantité est la quantité d'acide présente au moment où débute la réaction avec la base.

La réaction de la base avec le sel de cérium peut se faire en continu, on entend par là une addition simultanée des réactifs dans le milieu réactionnel. Cette réaction peut se faire sous air ou dans une atmosphère d'air et d'azote ou encore sous azote.

Le pH du milieu réactionnel est habituellement compris entre 7,5 et 9,5.

On obtient à l'issue de la réaction précitée un précipité. Ce précipité peut être séparé du milieu liquide par tout procédé connu par exemple par centrifugation. Le précipité ainsi obtenu peut ensuite être remis en suspension dans l'eau de manière à donner la dispersion de l'invention.

Avantageusement, le précipité issu de la réaction peut être lavé. Ce lavage peut se faire en remettant dans l'eau le précipité puis, après agitation, en séparant le solide du milieu liquide par centrifugation par exemple. Cette opération peut être répétée plusieurs fois si nécessaire.

La dispersion obtenue après remise en suspension dans l'eau est ensuite purifiée par ultrafiltration.

Si nécessaire, la dispersion peut être en outre concentrée par ultrafiltration par exemple.

Le lavage et l'ultrafiltration peuvent se faire sous air ou dans une atmosphère d'air et d'azote ou encore sous azote. L'atmosphère sous laquelle se déroulent ces opérations joue un rôle dans la transformation du cérium III en cérium IV.

Dans le cas d'une dispersion partiellement ou totalement en milieu solvant différent de l'eau, cette dispersion peut être préparée à partir de la dispersion aqueuse par addition du solvant organique à la dispersion aqueuse puis distillation pour éliminer l'eau ou traitement par une membrane d'ultra-filtration pour l'élimination progressive de l'eau.

Les dispersions de l'invention peuvent être utilisées dans de nombreuses applications. On peut citer la catalyse notamment pour post combustion automobile, dans ce cas les dispersion sont utilisées dans la préparation de catalyseurs. Les dispersions peuvent aussi être employées pour la lubrification, dans les céramiques, la fabrication de composés luminophores. Les dispersions peuvent aussi être mises en oeuvre pour leurs propriétés anti-UV par exemple dans la préparation de films de polymères (du type acrylique ou polycarbonate par exemple) ou de compositions cosmétiques notamment dans la préparation de crèmes anti-UV. Elles peuvent être utilisées enfin sur un substrat en tant qu'agents d'anticorrosion.

Des exemples vont maintenant être donnés. Dans ces exemples, la conductivité est mesurée à !'aide d'un conductimètre de type CDM 83 (Radiometer Copenhaguen) et de sa cellule de mesure de type CDC 304.

### EXEMPLE 1

Cet exemple concerne la préparation d'une dispersion de cérium à partir d'un mélange d'acétate et de chlorure de cérium et d'acide chlorhydrique.

On additionne dans un bécher 334 g d'acétate de cérium (III) à 49,29% en oxyde CeO₂ (Soit 0,957 mole de Ce) et 89,9 g de CeCl₃ à 45,90% en CeO₂ ( soit 0,24 mole de Ce), puis 357 g d'acide chlorhydrique concentré à 36% (soit 3,52 mole d'HCl) préalablement dilué par addition de 500 ml d'eau déminéralisée. On met sous agitation. On complète ensuite à 2000ml par de l'eau déminéralisée. L'ensemble est mis sous agitation jusqu'à l'obtention d'une solution limpide à l'oeil. Le mélange obtenu présente alors une concentration d'environ 0,6 M en Ce et un rapport (H/Ce) molaire de 2,9.

La précipitation du solide est réalisée dans un montage en continu comprenant:
- un réacteur d'un litre équipé d'un agitateur à pales, réglé à 400 t/mn avec un pied de cuve initial d'eau de 0,5 l et d'une électrode asservie à une pompe régulatrice de pH dont la consigne est fixée à un pH de 8,7;
- deux flacons d'alimentation contenant d'une part, la solution de sels de cérium précédemment décrite et d'autre part, une solution d'ammoniaque 10 N.

Le débit de la solution d'acétate de cérium est fixée à environ 500 ml/mn et le débit d'ammoniaque est asservie à la régulation de pH. Ainsi, 2000 ml du mélange de sels de cérium et 600 ml d'ammoniaque 10 N ont été additionnés en 243 mn.

Le rendement de précipitation en Ce est déterminé de l'ordre de 91%. Le précipité est séparé des eaux mères par centrifugation à 4500 tr/mn durant 10 mn. Sur une aliquote, par calcination à 1000°C, on détermine un pourcentage de 27,7% en oxyde CeO₂.

Le précipité est dispersée par addition d'eau déminéralisée pour obtenir une dispersion à 0,25M en Ce. On met sous agitation 15 mn. On centrifuge de nouveau. Deux opérations successives sont ainsi réalisées.

100 ml de la dispersion à 0,25 M en Ce sont dilués à 300 ml par de l'eau déminéralisée. Par ultrafiltration sur des membranes 3 KD, on concentre jusqu'à 100 ml. Trois ultrafiltrations sont ainsi réalisées. Une concentration supplémentaire est alors réalisée par ultrafiltration jusqu'à 34 ml lors de cette dernière ultrafiltration.

Par évaporation et calcination à 1000°C d'une aliquote bien déterminée de la dispersion obtenue, on mesure une concentration en CeO₂ de la dispersion finale obtenue de 60g/l.

La dispersion colloïdale présente un aspect limpide. Le pH de la dispersion est de 5. La teneur en NO₃ est inférieure à 80ppm. Le rapport CeIII/Ce total est de 1,8% (mesuré par méthode chimique) et la conductivité de la dispersion est de 0,7mS/cm.

### EXEMPLE 2

Cet exemple concerne la préparation d'une dispersion de cérium à partir d'acétate de cérium et d'acide acétique.

On additionne dans un bécher 416,5 g d'acétate de cérium (III) à 49,29% en oxyde CeO₂ (soit 1,19 mole de Ce), puis 144 g d'acide acétique concentré (soit 2,4 mole de CH₃COOH) préalablement dilué par addition de 100 ml d'eau déminéralisée. On met sous agitation. On complète ensuite à 2400ml par de l'eau déminéralisée. L'ensemble est mis sous agitation jusqu'à l'obtention d'une solution limpide à l'oeit. Le mélange obtenu présente alors une concentration d'environ 0,5M en Ce et un rapport (H/Ce) molaire de 2.

La précipitation du solide est réalisée dans le montage en continu décrit à l'exemple 1.

Ainsi, 2400 ml de la solution d'acétate de cérium et 2120 ml d'ammoniaque 3 N ont été additionnés en 270 mn.

Le rendement de précipitation en cérium est déterminé de l'ordre de 95,7%.

Le précipité est séparé des eaux mères par centrifugation à 4500 tlmn durant 10 mn. Sur une aliquote, par calcination à 1000°C, on détermine un pourcentage en CeO₂ du précipité de 23,4% en oxyde CeO₂.

Le précipité est dispersée par addition d'eau déminéralisée pour obtenir une dispersion à 0,25M en Ce. On met sous agitation 15 mn. On centrifuge de nouveau. Deux opérations successives sont ainsi réalisées.

100 ml de la dispersion à 0,25 M en Ce sont dilués à 300 ml par de l'eau déminéralisée et laissés sous agitation une nuit sous atmosphère d'air. Par ultrafiltration sur des membranes 3 KD, on concentre jusqu'à 100 ml. Trois ultrafiltrations sont ainsi réalisées. Une concentration supplémentaire est alors réalisée par ultrafiltration jusqu'à 50 ml lors de cette dernière ultrafiltration.

Par évaporation et calcination à 1000°C d'une aliquote bien déterminée de la dispersion obtenue, on mesure une concentration en CeO₂ de la dispersion finale obtenue de 57g/l.

La dispersion colloïdale présente un aspect limpide. Le pH de la dispersion est de 4,7.

La teneur en NO₃ est inférieure à 80 ppm. Le rapport CeIII/Ce total est de 1,9% et la conductivité de la dispersion est de 0,9mS/cm. La taille des colloïdes est de 3nm.

### EXEMPLE 3

Cet exemple illustre les propriétés anti-UV d'une dispersion de l'invention. On procède par mesures de transmission directe en fonction de la longueur d'onde.

Ces mesures de transmission sont réalisées sur un spectro-photomètre UV-Visible Perkin Elmer λ 900, équipée d'une cellule dont les parois sont en quartz et l'épaisseur de l'échantillon est de 2 mm. Les valeurs de transmission directe sont déterminées en mesurant l'intensité transmise, par rapport à une référence (mesures sur cuve sans dispersion colloïdale).

Une aliquote de la dispersion colloïdale est diluée par de l'eau déminéralisée jusqu'à obtenir une concentration finale de 3% massique en CeO₂.

La courbe de transmission obtenue est caractérisée par les valeurs suivantes :
A 400 nm, 10 % de transmission
A 418 nm, 50% de transmission
A 455 nm, 80% de transmission.

## Revendications

1. Dispersion colloïdale d'un composé de cérium, **caractérisée en ce qu'**elle présente une concentration en oxyde de cérium d'au moins 50g/l, un taux de cérium III par rapport au cérium total d'au moins 0,5%, une conductivité d'au plus 5mS/cm, un pH compris entre 4 et 8 et **en ce qu'**elle est constituée de particules présentant un diamètre moyen d'au plus 15nm.

2. Dispersion colloïdale d'un composé de cérium, **caractérisée en ce qu'**elle présente une concentration en oxyde de cérium d'au moins 50g/l, un taux de cérium III par rapport au cérium total d'au moins 0,5%, une conductivité d'au plus 5mS/cm, un pH compris entre 4 et 8 et **en ce qu'**elle est constituée de particules présentant un diamètre moyen compris entre 2nm et 6nm.

3. Dispersion selon la revendication 1 ou 2, **caractérisée en ce qu'**elle présente un taux de cérium III d'au moins 1%, plus particulièrement d'au moins 1,5%.

4. Dispersion selon l'une des revendications précédentes, **caractérisée en ce qu'**elle présente une teneur en anion nitrates inférieure à 80ppm.

5. Dispersion selon l'une des revendications précédentes, **caractérisée en ce qu'**elle présente une conductivité d'au plus 2mS/cm, plus particulièrement d'au plus 1mS/cm.

6. Dispersion selon l'une des revendications 1 à 4, **caractérisée en ce qu'**elle présente une conductivité inférieure à 0,3mS/cm.

7. Dispersion selon l'une des revendications précédentes, **caractérisée en ce que** la phase continue est l'eau.

8. Dispersion selon l'une des revendications 1 à 6, **caractérisée en ce que** la phase continue est constituée par un mélange eau/solvant miscible à l'eau ou par un solvant organique.

9. Procédé de préparation d'une dispersion colloïdale selon l'une des revendications précédentes, **caractérisé en ce qu'**on fait réagir au moins un sel de cérium III avec une base et en présence d'un acide en quantité telle que le rapport molaire H⁺/Ce soit supérieur à 1, puis on redisperse dans l'eau le précipité issu de la réaction précédente et on purifie par ultrafiltration la dispersion obtenue.

10. Procédé selon la revendication 9, **caractérisé en ce que** le sel de cérium III est un acétate ou un chlorure.

11. Procédé selon l'une des revendications 9 ou 10, **caractérisé en ce que** l'acide précité est l'acide acétique ou l'acide chlorhydrique.

12. Procédé selon l'une des revendications 9 à 11, **caractérisé en ce qu'**on effectue la réaction en continu.

13. Procédé pour la préparation d'une dispersion colloïdale dont la phase continue est constituée par un mélange eau/solvant miscible à l'eau ou par un solvant organique, **caractérisé en ce qu'**on ajoute le solvant organique à la dispersion aqueuse obtenue par le procédé selon l'une des revendications 9 à 12 puis on distille pour éliminer l'eau, ou on traite la dispersion par une membrane d'ultra-filtration pour l'élimination progressive de l'eau.

14. Utilisation d'une dispersion du type selon l'une des revendications 1 à 8 ou du type obtenu par le procédé selon l'une des revendications 9 à 13, sur un substrat comme agent anticorrosion, dans la préparation de films de polymères, dans une composition cosmétique, en catalyse notamment pour post combustion automobile, en lubrification ou dans les céramiques.

## Claims

1. A colloidal dispersion of a cerium compound, **characterized in that** its cerium oxide concentration is at least 50 g/l, its cerium III content with respect to the total cerium content is at least 0.5%, its conductivity is at most 5 mS/cm, its pH is in the range 4 to 8, and **in that** it is constituted by particles with a mean diameter of at most 15 nm.

2. A colloidal dispersion of a cerium compound, **characterized in that** its cerium oxide concentration is at least 50 g/l, its cerium III content with respect to the total cerium content is at least 0.5%, its conductivity is at most 5 mS/cm, its pH is in the range 4 to 8, and **in that** it is constituted by particles with a mean diameter in the range 2 run to 6 nm.

3. A dispersion according to claim 1 or claim 2, **characterized in that** its cerium III content is at least 1 %, more particularly at least 1.5%.

4. A dispersion according to one of the preceding claims, **characterized in that** its nitrate anion content is less than 80 ppm.

5. A dispersion according to one of the preceding claims, **characterized in that** its conductivity is at most 2 mS/cm, more particularly at most 1 mS/cm.

6. A dispersion according to one of claims 1 to 4, **characterized in that** its conductivity is less than 0.3 mS/cm.

7. A dispersion according to one of the preceding claims, **characterized in that** the continuous phase is water.

8. A dispersion according to any of claims 1 to 6, **characterized in that** the continuous phase is constituted by a water/water-miscible solvent mixture or by an organic solvent.

9. A process for preparing a colloidal dispersion according to one of the preceding claims, **characterized in that** at least one cerium III salt is reacted with a base in the presence of an acid in an amount such that the H⁺/Ce mole ratio is more than 1, then the precipitate from the preceding reaction is re-dispersed in water and the dispersion obtained is purified by ultrafiltration.

10. A process according to claim 9, **characterized in that** the cerium III salt is an acetate or a chloride.

11. A process according to claim 9 or claim 10, **characterized in that** said acid is acetic acid or hydrochloric acid.

12. A process according to one of claims 9 to 11, **characterized in that** the reaction is carried out in a continuous process.

13. A process for preparing a colloidal dispersion wherein the continuous phase is constituted by a water/water-miscible solvent mixture or by an organic solvent, **characterized in that** the organic solvent is added to the aqueous dispersion obtained by a process according to any one of claims 9 to 12 then distilled to eliminate water, or the dispersion is treated with an ultrafiltration membrane to progressively eliminate water.

14. Use of a dispersion of the type defined in one of claims 1 to 8 or of the type obtained by the process according to one of claims 9 to 13, on a substrate as an anti-corrosion agent, in preparing polymer films, in a cosmetic composition, in catalysis, in particular for automobile post combustion, in lubricants or in ceramics.

## Patentansprüche

1. Kolloidale Dispersion einer Cer-Verbindung, **dadurch gekennzeichnet, dass** sie eine Konzentration an Ceroxid von mindestens 50 g/l, ein Verhältnis von Cer-III zu Cer-Gesamt von mindestens 0,5 %, eine Leitfähigkeit von höchstens 5 mS/cm und einen pH zwischen 4 und 8 aufweist und aus Teilchen zusammengesetzt ist, die einen mittleren Durchmesser von höchstens 15 nm aufweisen.

2. Kolloidale Dispersion einer Cer-Verbindung, **dadurch gekennzeichnet, dass** sie eine Konzentration an Ceroxid von mindestens 50 g/l, ein Verhältnis von Cer-III zu Cer-Gesamt von mindestens 0,5 %, eine Leitfähigkeit von höchstens 5 mS/cm und einen pH zwischen 4 und 8 aufweist und aus Teilchen zusammengesetzt ist, die einen mittleren Durchmesser zwischen 2 nm und 6 nm aufweisen.

3. Dispersion gemäß Anspruch 1 oder 2, **dadurch gekennzeichnet, dass** sie ein Verhältnis von Cer-III von mindestens 1 % aufweist, insbesondere mindestens 1,5 %.

4. Dispersion gemäß einem der vorhergehenden Ansprüche, **dadurch gekennzeichnet, dass** sie einen Nitratanionengehalt von weniger als 80 ppm aufweist.

5. Dispersion gemäß einem der vorhergehenden Ansprüche, **dadurch gekennzeichnet, dass** sie eine Leitfähigkeit von höchstens 2 mS/cm aufweist, insbesondere höchstens 1 mS/cm.

6. Dispersion gemäß einem der Ansprüche 1 bis 4, **dadurch gekennzeichnet, dass** sie eine Leitfähigkeit von weniger als 0,3 mS/cm aufweist.

7. Dispersion gemäß einem der vorhergehenden Ansprüche, **dadurch gekennzeichnet, dass** die kontinuierliche Phase Wasser ist.

8. Dispersion gemäß einem der Ansprüche 1 bis 6, **dadurch gekennzeichnet, dass** die kontinuierliche Phase aus einer Mischung Wasser/mit Wasser mischbarem Lösungsmittel oder einem organischen Lösungsmittel zusammengesetzt ist.

9. Verfahren zur Herstellung einer kolloidalen Dispersion gemäß einem der vorhergehenden Ansprüche, **dadurch gekennzeichnet, dass** man wenigstens ein Cer-III-Salz mit einer Base und in Gegenwart einer Säure in einer solchen Menge, dass das Molverhältnis H⁺/Ce größer als 1 ist, umsetzt, anschließend den aus der vorhergehenden Reaktion stammenden Niederschlag erneut in Wasser dispergiert und die erhaltene Dispersion mittels Ultrafiltration reinigt.

10. Verfahren gemäß Anspruch 9, **dadurch gekennzeichnet, dass** das Cer-III-Salz ein Acetat oder ein Chlorid ist.

11. Verfahren gemäß einem der Ansprüche 9 oder 10, **dadurch gekennzeichnet, dass** die vorgenannte Säure Essigsäure oder Chlorsäure ist.

12. Verfahren gemäß einem der Ansprüche 9 bis 11, **dadurch gekennzeichnet, dass** die Reaktion kontinuierlich durchgeführt wird.

13. Verfahren zur Herstellung einer kolloidalen Dispersion, deren kontinuierliche Phase aus einer Mischung Wasser/mit Wasser mischbarem Lösungsmittel oder einem organischen Lösungsmittel zusammengesetzt ist, **dadurch gekennzeichnet, dass** man das organische Lösungsmittel zu der wässrigen Dispersion zugibt, die nach dem Verfahren gemäß einem der Ansprüche 9 bis 12 erhalten worden ist, und anschließend destilliert, um das Wasser zu entfernen, oder die Dispersion mittels einer Ultrafiltrationsmembran behandelt, um das Wasser fortschreitend zu entfernen.

14. Verwendung einer Dispersion der Art gemäß einem der Ansprüche 1 bis 8 oder der Art, die nach dem Verfahren gemäß einem der Ansprüche 9 bis 13 erhalten wird, auf einem Substrat als Korrosionsschutzmittel bei der Herstellung von Polymerfilmen, in einer kosmetischen Zubereitung, bei der Katalyse, insbesondere für Automobil-Nachverbrennung, bei der Schmierung oder in Keramiken.
